# EUROPEAN PATENT SPECIFICATION

(11) **EP 2 779 911 B1**
(45) Date of publication and mention of the grant of the patent: **26.04.2017**
(21) Application number: 12808542.0
(22) Date of filing: 14.11.2012
(51) Int. Cl.: A61B 17/00, A61B 17/34

(54) **VESSEL PROTECTION MEMBRANE**
GEFÄSSSCHUTZMEMBRAN
MEMBRANE DE PROTECTION DE VAISSEAU

(30) Priority: 15.11.2011 US 201161559907 P; 13.11.2012 US 201213675375
(43) Date of publication of application: 24.09.2014
(73) Proprietor: Boston Scientific Scimed, Inc., Maple Grove, MN 55311-1566 (US)
(72) Inventor: WEBER, Jan, 6228 GJ, Maastricht (NL); FLANAGAN, Aiden, Kilcolgan, Co. Galway (IE)
(74) Representative: Peterreins Schley
(86) International application number: PCT/US2012/065089
(87) International publication number: WO 2013/074679

(56) References cited:
- US-A1- 2002 115 982
- US-A1- 2002 138 041
- US-A1- 2005 085 842
- US-A1- 2007 149 998

## Description

### Technical Field

The invention relates generally to medical devices and more particularly to medical devices that are adapted for use in percutaneous medical procedures.

### Background

Some percutaneous procedures can involve relatively large, bulky medical devices that must be advanced through relatively narrow and tortuous vasculature. Such advancement may result in peripheral damage to the wall of the vessel, particularly when the medical device must traverse a sharp bend in the vessel, due to the shear force exerted by the medical device against the vessel wall. A continuing need exists to reduce or eliminate the chances of injuring the vessel during percutaneous medical procedures.

US 2005/00852842 discloses a collapsible/expandable guide for delivering fluids and/or instruments.

### Summary

The present invention is directed to a medical device assembly as set forth in the claims. The medical device assembly includes an elongated guidewire, an elongated highly flexible tubular membrane disposed about the guidewire, the membrane having a wall defining a lumen through the membrane, the lumen having a first inner diameter, and a percutaneous medical device having a maximum outer diameter greater than the first inner diameter, wherein the membrane is configured to permit the percutaneous medical device to pass through the lumen. The medical device assembly according to the invention further comprises a straightening wire (50) slidably disposed in a passage formed within the wall (22) of the membrane (20), wherein the passage is oriented generally parallel to the lumen.

Although discussed with specific reference to use within the vasculature of a patient, medical devices in accordance with the disclosure can be adapted and configured for use in other parts of the anatomy, such as the digestive system, the respiratory system, or other parts of the anatomy of a patient.

The invention is defined in claim 1. Further embodiments of the invention are defined in the dependent claims.

### Brief Description of the Figures

FIG. 1 is a partial schematic view of an example membrane disposed within a vessel;
FIG. 1A is a cross-sectional view of FIG. 1 taken along the line 1A-1A;
FIG. 2 is a partial schematic view of the example membrane of FIG. 1 including a medical device disposed therein;
FIG. 2A is a cross-sectional view of FIG. 2 taken along the line 2A-2A;
FIG. 2B is a cross-sectional view of FIG. 2 taken along the line 2B-2B;
FIG. 2C is a cross-sectional view of FIG. 2 taken along the line 2C-2C;
FIG. 3 is a perspective view of a portion of the example membrane of FIG. 1;
FIG. 4 is a perspective view of a portion of the example membrane of FIG. 1 including a straightening wire;
FIG. 4A is a schematic view of the straightening wire of FIG. 4 in an extended configuration;
FIG. 4B is a schematic view of the straightening wire of FIG. 4 in a partially contracted configuration;
FIG. 4C is a schematic view of the straightening wire of FIG. 4 in a contracted configuration;
FIG. 5 is a perspective view of a portion of the example membrane of FIG. 3 including circumferential fibers; and
FIG. 5A is a partial cut-away view of the example membrane of FIG. 5.

While the invention is amenable to various modifications and alternative forms, specifics thereof have been shown by way of example in the drawings and will be described in greater detail below. It should be understood, however, that the intention is not to limit the invention to the particular embodiments described.

### Detailed Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the term "about" may include numbers that are rounded to the nearest significant figure.

Weight percent, percent by weight, wt %, wt-%, % by weight, and the like are synonyms that refer to the concentration of a substance as the weight of that substance divided by the weight of the composition and multiplied by 100.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawings wherein like reference numerals indicate like elements throughout several views. The detailed description and drawings are intended to illustrate but not limit the claimed invention. Some percutaneous medical procedures may require relatively large and/or bulky medical devices to be inserted through a patient's vasculature. In some cases, those medical devices may pass through a tortuous and/or peripheral vessel. As the medical device is navigated through the vasculature, the vessel wall may be subjected to a shear force applied by the medical device as the medical device moves through the vessel lumen and makes contact with the vessel wall. The shear force may cause injury to the vessel in tortuous portions of the vasculature as the medical device is forced to make turns, or when the medical device travels through calcified or diseased vessels.

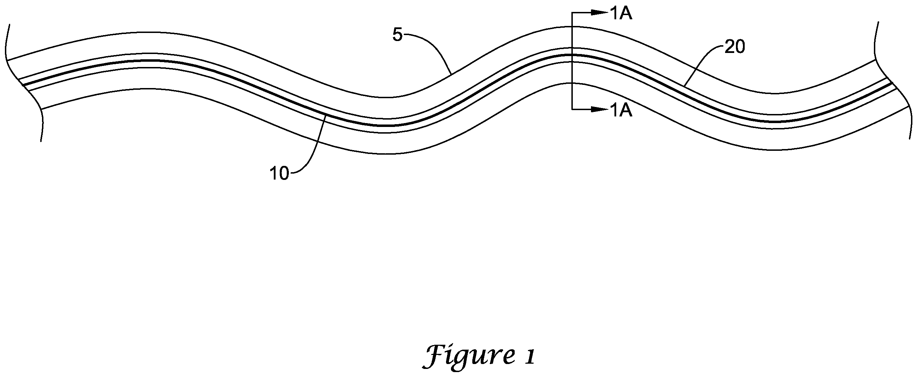

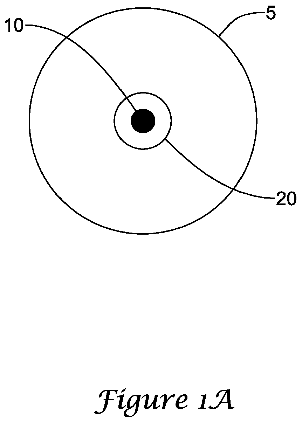

The risk of injury from the shear force applied against the vessel wall by a traversing medical device may be reduced by protecting the vessel wall from the shear force. FIG. 1 illustrates a schematic view of a vessel 5 having a guidewire 10 disposed therein. An example membrane 20, which may be an elongated tubular membrane made from a highly flexible elastic material, or having a highly flexible elastic structure, is shown disposed about the guidewire 10.

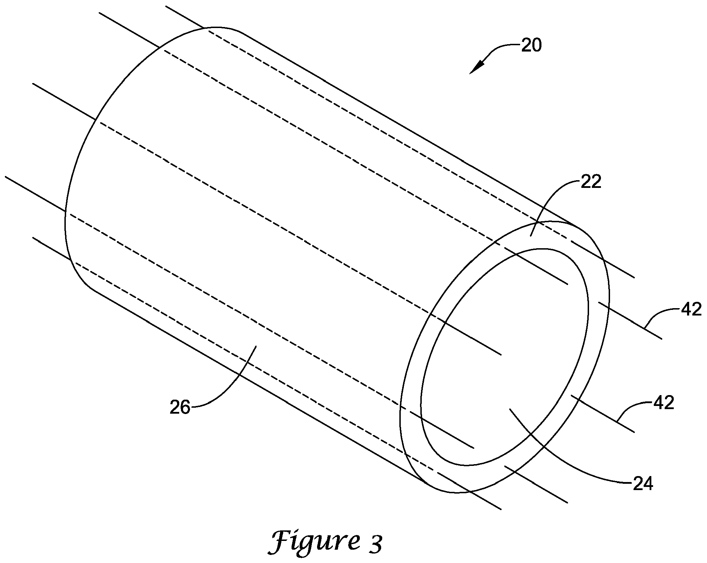

Some structural details of an example membrane 20 may be seen in FIGS. 3-5A. An example membrane 20 in accordance with the invention may include none, one, some, or all of the features shown in FIGS. 3-5A. In general, the membrane 20 may be described as having an elongated tubular structure. The membrane 20 may include a wall 22 having an inner surface 24 and an outer surface 26. In some embodiments, the wall 22 may be defined by the inner surface 24 and the outer surface 26, and may form an annular tube, although other shapes or configurations are contemplated.

The membrane 20 and/or the lumen may be configured to radially expand and/or contract between a relaxed condition and an expanded condition. In the relaxed condition, the lumen may have a first inner diameter defined by the inner surface 24 of the wall 22. In the expanded condition, the lumen may have a second inner diameter defined by the inner surface 24 of the wall 22. In some embodiments, the second inner diameter may be greater than the first inner diameter. Similarly, the membrane 20 may have an outer diameter defined by the outer surface 26 of the wall 22. In the relaxed condition, the membrane 20 may have a first outer diameter defined by the outer surface 26 of the wall 22. In the expanded condition, the membrane 20 may have a second outer diameter defined by the outer surface 26 of the wall 22. In some embodiments, the second outer diameter may be greater than the first outer diameter.

In some embodiments, the membrane 20 may be configured to permit the lumen to radially expand to the second inner diameter. In some embodiments, the membrane 20 is configured to substantially prevent axial stretching along the lumen. In other words, the membrane 20 may permit the lumen to expand radially outward without stretching or expanding in an axial direction. To facilitate this behavior or characteristic, the membrane 20 may be formed to include a first plurality of filaments or fibers 42 oriented axially, or generally parallel to the lumen or a central axis of the lumen and/or membrane 20, as shown in FIGS. 3-5. In some embodiments, the first plurality of filaments or fibers 42 may each be longitudinally aligned with the lumen. In some embodiments, the first plurality of filaments or fibers 42 may be embedded within the wall 22 of the membrane 20. Although not expressly illustrated, in some embodiments, the first plurality of filaments or fibers 42 may be disposed on the inner surface 24 or on the outer surface 26 of the wall 22. The first plurality of filaments or fibers 42 may exhibit high non-compliance in an axial direction, and may be highly compliant or flexible in a tangential (i.e., transverse or radial) direction relative to the central longitudinal axis of the lumen.

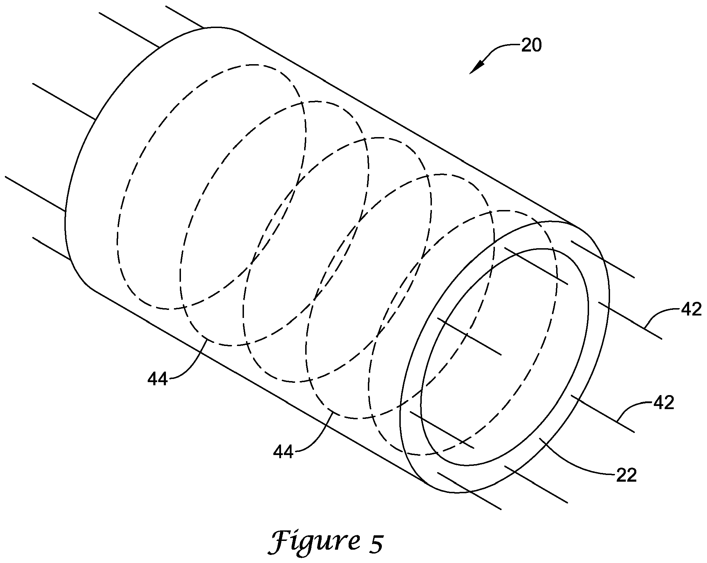

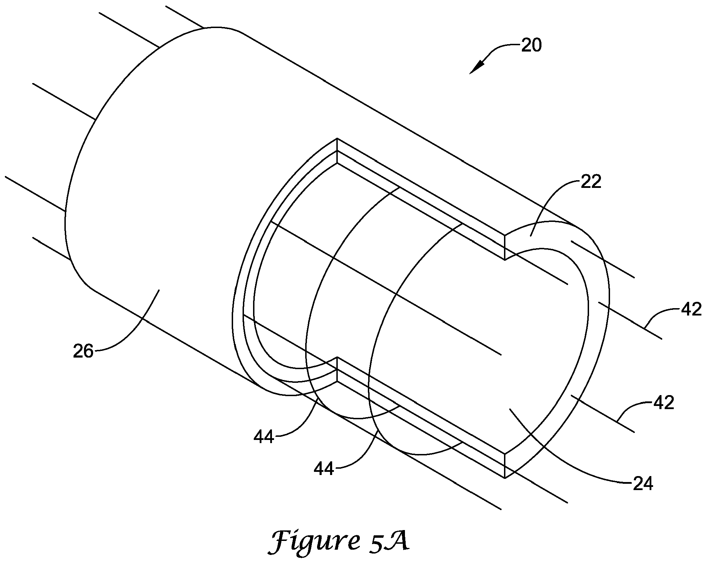

In some embodiments, the membrane 20 may be configured to expand to no more than a predetermined maximum second outer diameter or at a predetermined maximum rate. In some embodiments, the membrane 20 may be configured to permit the lumen to radially expand to no more than a predetermined maximum second inner diameter. To facilitate this behavior or characteristic, the membrane 20 may be formed to include a second plurality of filaments or fibers 44 generally oriented circumferentially about the lumen or a central axis of the lumen and/or membrane 20, as shown in FIGS. 5 and 5A. In some embodiments, the second plurality of filaments or fibers 44 may be elastic or semi-elastic, such that a limited amount or rate of stretching may occur in the radial direction. In some embodiments, the second plurality of filaments or fibers 44 may each be curved about the lumen or form an annular shape about the lumen and/or the central axis. In some embodiments, the second plurality of filaments or fibers 44 may be embedded within the wall 22 of the membrane 20, as seen in FIGS. 5 and 5A. Although not expressly illustrated, in some embodiments, the second plurality of filaments or fibers 44 may be disposed on the inner surface 24 or on the outer surface 26 of the wall 22. The second plurality of filaments or fibers 44 may serve to limit the radial expansion of the membrane 20 and/or the lumen to a predetermined maximum value. In limiting the radial expansion, radially outward force and/or the shear force created by the medical device 30 against the vessel 5 by the outer surface 26 of the membrane 20 as the medical device 30 passes therethrough may be reduced, or eliminated altogether.

In some embodiments, the inner surface 24 of the wall 22 may include one or more layers or coating, such as a lubricious coating, a hydrophilic coating, a hydrophobic coating, or other suitable coatings, and the like, or the membrane 20 may include a lubricant disposed within the lumen. In some embodiments, the outer surface 26 of the wall 22 may include one or more layers or coatings, such as a lubricious coating, a hydrophilic coating, a hydrophobic coating, or other suitable coating, and the like, or the membrane 20 may include a lubricant disposed upon the outer surface 26.

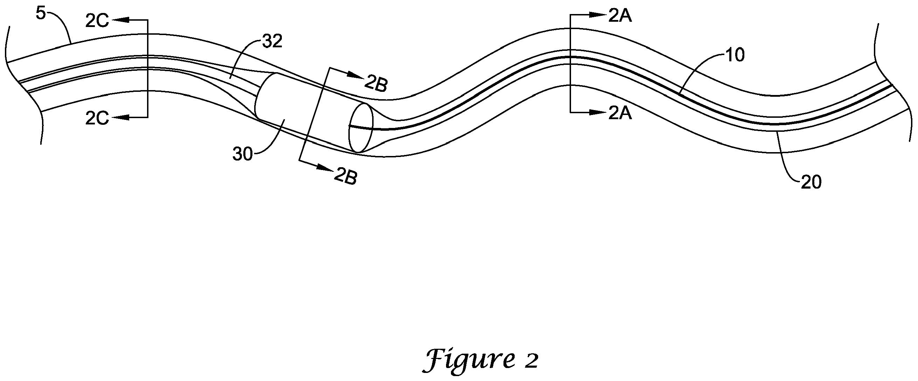

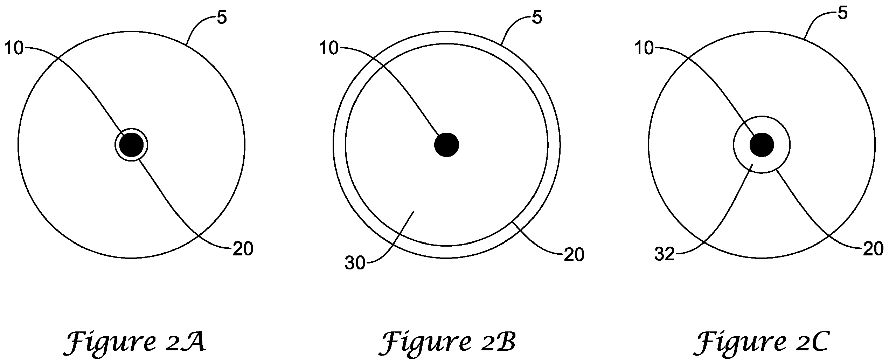

FIG. 2 illustrates a schematic view of an example membrane 20 disposed within a vessel 5 having a medical device 30 being advanced through a lumen of the membrane 20. For reference, the view shown in FIG. 2 may generally be described as showing "proximal" toward the left side of FIG. 2, and "distal" toward the right side of FIG. 2. Accordingly, the medical device 30 is shown as being advanced from the proximal end or portion of the membrane 20 toward the distal end or portion of the membrane 20. However, the skilled artisan will appreciate that the orientations and/or directions may be reversed as necessary or appropriate.

The medical device 30 is generally shown schematically in FIG. 2 as a cylinder disposed at the distal end of an elongate shaft 32. One of ordinary skill in the art will recognize that the medical device 30 and the elongate shaft 32 may be a single object, element, or device, or may be a combination of elements that together make up a medical device suitable for insertion through the membrane 20. For example, the medical device 30 may be a single catheter or sheath having a uniform outer diameter or the medical device 30 may be an assembly having a stepped or variable outer diameter, or the medical device 30 may be some combination of these (i.e., a single catheter with a stepped or variable outer diameter).

In some embodiments, the medical device 30 may include an atherectomy device, an angioplasty device, a balloon dilatation catheter, a distal protection device, an embolic filtering device, a valvectomy device, a valvuloplasty device, a stent delivery device, a transaortic valve implantation device, an ablation device, an object retrieval device, a guide catheter or sheath, a diagnostic catheter, or other suitable device. For simplicity, the following discussion will generally refer to a medical device 30, which may or may not include the elongate shaft 32 shown in FIG. 2.

The medical device 30 may have a maximum outer diameter that may be defined as the farthest or largest radial extent from a central longitudinal axis of the medical device 30, or the maximum circumference or perimeter of the medical device 30. In some embodiments, the medical device 30 may have a maximum outer diameter that is greater than the first inner diameter of the lumen of the membrane 20 (i.e., the inner diameter of the lumen of the membrane 20 in the radially relaxed condition) and/or the first outer diameter of the membrane 20 (i.e., the outer diameter of the membrane 20 in the radially relaxed condition). During operation, the membrane 20 may be advanced through the vessel 5 to a treatment site. In some embodiments, the membrane 20 may be delivered via a guide or delivery catheter, or the membrane 20 may be fixedly or removably attached to a guidewire for navigation through the vasculature to the treatment site. For example, the membrane 20 may be attached along one side to a guidewire, or the membrane 20 may be attached at its distal end to a distal end of a guidewire. The guidewire may push or pull the membrane 20 through the vasculature to the treatment site. In some embodiments, the elastic nature of the membrane 20 may form a natural fit to the guidewire. In some embodiments, the straightening wire 50, which is described further below, may act as a pusher or guide for the membrane 20 during navigation or advancement through the vasculature to the treatment site.

Following placement within the vessel 5, the membrane 20 may remain or be maintained in a substantially stationary position along the guidewire 10 or relative to the vessel 5 as the medical device 30 is passed through the lumen. In other words, as the medical device 30 is advanced distally, the membrane 20 does not move axially within the vessel 5. The membrane 20 is configured to permit the lumen to radially expand around the medical device 30 as the medical device 30 is advanced through the lumen, as shown in FIGS. 2 and 2B. As the medical device 30 is advanced, the membrane 20 permits the medical device 30 to push the wall 22 radially outward from a central axis of the lumen such that the medical device 30 is permitted to pass through the lumen. In some embodiments, the membrane 20 is configured to permit the lumen to radially expand to a second inner diameter equal to or greater than the maximum outer diameter of the medical device 30. In some embodiments, the membrane 20 is configured to radially expand to a second outer diameter greater than the maximum outer diameter of the medical device 30.

Once the maximum outer diameter of the medical device 30 has moved past a particular axial position along the membrane, the membrane will then constrict inwards toward the central longitudinal axis behind the medical device 30, as shown in FIGS. 2 and 2C. In general, the lumen will maintain a shape or profile that closely conforms to that of the medical device 30 passing therethrough. Accordingly, the membrane 20 may exhibit elasticity in a radial direction with respect to the diameter of the membrane 20 and/or the lumen.

Additionally, while not expressly illustrated, in some embodiments, the membrane 20 may be formed as a mesh, a braid, or a thin-film membrane having a plurality of openings and/or apertures extending laterally or transversely through the wall 22. The plurality of openings and/or apertures extending through the wall 22 may permit the membrane 20 to contract in the relaxed condition to an even smaller first outer diameter, thereby facilitating use in more tortuous vasculature than a membrane 20 lacking the plurality of openings and/or apertures, while retaining the ability to protect the wall of the vessel 5 from undesirable contact, friction, and shear forces.

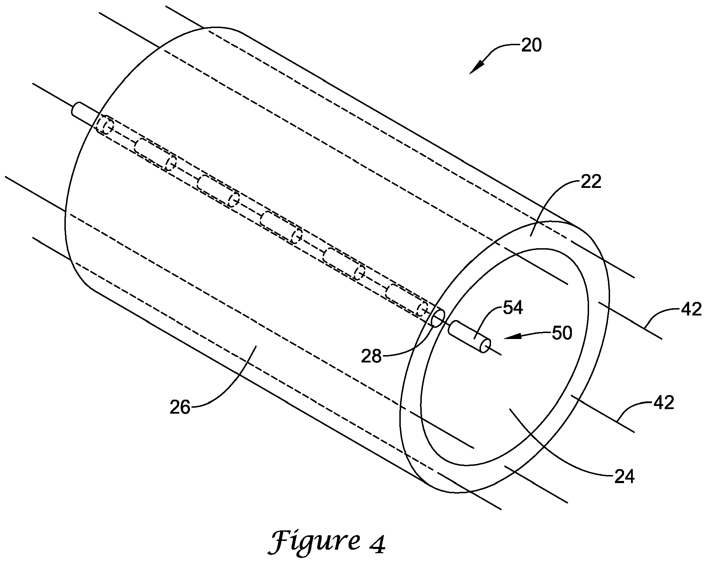

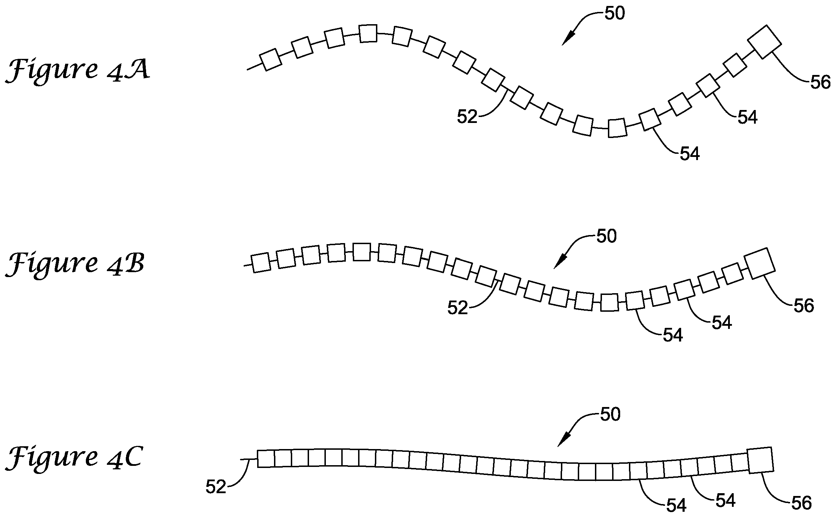

In some embodiments, the membrane 20 may include a straightening wire 50. The straightening wire 50 may include a thin, flexible central wire 52 having a fixed stop or protrusion 56 at a distal end thereof. The straightening wire 50 may include a plurality of cylindrical (or other shaped) tubular elements 54 disposed thereon proximal of the fixed stop 56. As shown in FIG. 4, the straightening wire 50 may be slidably disposed within a passage 28 formed within the wall 22. The passage 28 may be embedded within the wall 22, or in other words, disposed between the inner surface 24 and the outer surface 26, and the passage may be oriented generally parallel to the lumen. In some embodiments, the straightening wire 50 and/or the passage 28 may extend longitudinally within the membrane 20.

The straightening wire 50 may be axially actuatable between a relatively flexible extended configuration and a relatively rigid contracted configuration. When the straightening wire 50 is extended or translated distally, the straightening wire assumes the extended configuration and the tubular elements 54 become spaced apart, as illustrated in FIG. 4A. In the extended configuration, the straightening wire 50 is generally very flexible, which aids in navigation through tortuous vasculature. When the central wire 52 is pulled or translated proximally, as shown in FIG. 4B, the distal stop 56 is translated proximally causing the tubular elements 54 to be moved closer together as the straightening wire 50 moves toward the contracted configuration. As the tubular elements 54 are translated closer together, the straightening wire 50 becomes stiffer and/or more rigid. In the contracted configuration, when the tubular elements 54 are translated a sufficient distance to bring them into end-to-end abutting contact, as shown in FIG. 4C, the straightening wire 50 is considerably more rigid than when the tubular elements 54 are spaced apart in the extended configuration. Accordingly, the straightening wire 50 may be used to selectively straighten the path of a tortuous vessel 5 (or a portion thereof) as a bulky medical device 30 is advanced therethrough to reduce the shear force that the medical device 30 exerts upon the wall of the vessel 5.

The guidewire 10 and/or the straightening wire 50 may be made from materials such as metals, metal alloys, polymers, metal-polymer composites, or other suitable materials, and the like. Some examples of suitable materials may include metallic materials such as stainless steels (e.g. 304v stainless steel or 316L stainless steel), nickel-titanium alloys (e.g., nitinol, such as super elastic or linear elastic nitinol), nickel-chromium alloys, nickel-chromium-iron alloys, cobalt alloys, nickel, titanium, platinum, or alternatively, a polymeric material, such as a high performance polymer, or other suitable materials, and the like. The word nitinol was coined by a group of researchers at the United States Naval Ordinance Laboratory (NOL) who were the first to observe the shape memory behavior of this material. The word nitinol is an acronym including the chemical symbol for nickel (Ni), the chemical symbol for titanium (Ti), and an acronym identifying the Naval Ordinance Laboratory (NOL).

The membrane 20 and/or the first or second plurality of fibers 42/44 may be made of any suitable material, for example, a polymeric material, a metal, a metal alloy, a metal-polymer composite, or the like. Examples of suitable polymers may include polyurethane, a polyetherester such as ARNITEL® available from DSM Engineering Plastics, a polyester such as HYTREL® available from DuPont, a linear low density polyethylene such as REXELL®, a polyamide such as DURETHAN® available from Bayer or CRISTAMID® available from Elf Atochem, an elastomeric polyamide, a block polyamide/ether, a polyether block amide such as PEBA available under the trade name PEBAX®, silicones, polyethylene, Marlex high-density polyethylene, polyetheretherketone (PEEK), polyimide (PI), and polyetherimide (PEI), a liquid crystal polymer (LCP) alone or blended with other materials.

Portions of the guidewire 10, the membrane 20, the medical device 30, and/or the straightening wire 50 may be made of, may be doped with, may include a layer of, or otherwise may include a radiopaque material. Radiopaque materials are understood to be materials capable of producing a relatively bright image on a fluoroscopy screen or another imaging technique such as X-ray during a medical procedure. This relatively bright image aids the user of device in determining its location. For example, one or more of the elements described above (i.e., the guidewire 10, the membrane 20, the medical device 30, etc.) may include or be formed from a radiopaque material. Suitable materials can include, but are not limited to, bismuth subcarbonate, iodine, gold, platinum, palladium, tantalum, tungsten or tungsten alloy, and the like.

It should be understood that although the above discussion was focused on percutaneous medical procedures within the vasculature of a patient, other embodiments or methods in accordance with the invention can be adapted and configured for use in other parts of the anatomy of a patient. For example, devices and methods in accordance with the invention can be adapted for use in the digestive or gastrointestinal tract, such as in the mouth, throat, small and large intestine, colon, rectum, and the like. For another example, devices and methods can be adapted and configured for use within the respiratory tract, such as in the mouth, nose, throat, bronchial passages, nasal passages, lungs, and the like. Similarly, the devices and methods described herein with respect to percutaneous deployment may be used in other types of surgical procedures as appropriate. For example, in some embodiments, the devices may be deployed in a non-percutaneous procedure. Devices and methods in accordance with the invention can also be adapted and configured for other uses within the anatomy.

It should be understood that this disclosure is, in many respects, only illustrative. Changes may be made in details, particularly in matters of shape, size, and arrangement of steps limited to, bismuth subcarbonate, iodine, gold, platinum, palladium, tantalum, tungsten or tungsten alloy, and the like.

It should be understood that although the above discussion was focused on percutaneous medical procedures within the vasculature of a patient, other embodiments or methods in accordance with the invention can be adapted and configured for use in other parts of the anatomy of a patient. For example, devices and methods in accordance with the invention can be adapted for use in the digestive or gastrointestinal tract, such as in the mouth, throat, small and large intestine, colon, rectum, and the like. For another example, devices and methods can be adapted and configured for use within the respiratory tract, such as in the mouth, nose, throat, bronchial passages, nasal passages, lungs, and the like. Similarly, the devices and methods described herein with respect to percutaneous deployment may be used in other types of surgical procedures as appropriate. For example, in some embodiments, the devices may be deployed in a non-percutaneous procedure. Devices and methods in accordance with the invention can also be adapted and configured for other uses within the anatomy.

It should be understood that this disclosure is, in many respects, only illustrative. Changes may be made in details, particularly in matters of shape, size, and arrangement of steps without exceeding the scope of the invention. The invention's scope is, of course, defined in the language in which the appended claims are expressed.

## Claims

1. A medical device assembly comprising:
an elongated guidewire (10);
an elongated highly flexible tubular membrane (20) disposed about the guidewire (10), the membrane (20) having a wall (22) defining a lumen through the membrane (20), the lumen having a first inner diameter; and
a percutaneous medical device (30) having a maximum outer diameter greater than the first inner diameter;
wherein the membrane (20) and the lumen is configured to radially expand and contract between a relaxed condition and an expanded condition so as to permit the percutaneous medical device (30) to pass through the lumen;
**characterized by** a straightening wire (50) slidably disposed in a passage formed within the wall (22) of the membrane (20), wherein the passage is oriented generally parallel to the lumen.

2. The medical device assembly of claim 1, wherein the membrane (20) is configured to permit the lumen to radially expand to a second inner diameter equal to or greater than the maximum outer diameter.

3. The medical device assembly of claim 1, wherein the membrane (20) remains in a substantially stationary position along the guidewire (10) as the percutaneous medical device (30) is passed through the lumen.

4. The medical device assembly of claim 1, wherein the membrane (20) defines an outer diameter that is less than the maximum outer diameter of the percutaneous medical device (30).

5. The medical device assembly of claim 1, wherein the membrane (20) includes a first plurality of fibers (42) each oriented parallel to the lumen.

6. The medical device assembly of claim 5, wherein the first plurality of fibers (42) is embedded within the wall (22) of the membrane (20).

7. The medical device assembly of claim 5, wherein the first plurality of fibers (42) is disposed on a surface of the wall (22) of the membrane (20).

8. The medical device assembly of claim 5, wherein the first plurality of fibers (42) substantially prevents axial stretching of the membrane (20) along the lumen.

9. The medical device assembly of claim 1, wherein the membrane (20) includes a second plurality of fibers (44) each oriented circumferentially about the lumen.

10. The medical device assembly of claim 9, wherein the second plurality of fibers (44) is embedded within the wall (22) of the membrane (20).

11. The medical device assembly of claim 9, wherein the second plurality of fibers (44) is disposed on a surface of the wall (22) of the membrane (20).

12. The medical device assembly of claim 9, wherein the second plurality of fibers (44) limits the radial expansion of the membrane and/or the lumen to a predetermined maximum value.

13. The medical device assembly of claim 1, wherein the straightening wire (50) is axially actuatable between a relatively flexible extended configuration and a relatively rigid contracted configuration.

14. The medical device assembly of claim 1, wherein the wall (22) includes a lubricious coating disposed on an inner surface (24) thereof.

15. The medical device assembly of claim 1, wherein the percutaneous medical device (30) is selected from the following: an atherectomy device, an angioplasty device, a balloon dilatation catheter, a distal protection device, an embolic filtering device, a valvectomy device, a valvuloplasty device, a stent delivery device, a transaortic valve implantation device, an ablation device, an object retrieval device, a guide catheter or sheath, or a diagnostic catheter.

## Patentansprüche

1. Medizinische Vorrichtungsanordnung, umfassend:
einen länglichen Führungsdraht (10),
eine längliche hochflexible röhrenförmige Membran (20), die um den Führungsdraht (10) angeordnet ist, wobei die Membran (20) eine Wand (22) hat, die ein Lumen durch die Membran (20) definiert, wobei das Lumen einen ersten inneren Durchmesser hat, und
eine perkutane medizinische Vorrichtung (30) mit einem maximalen äußeren Durchmesser, der größer als der erste innere Durchmesser ist,
wobei die Membran (20) und das Lumen dazu konfiguriert sind, zwischen einem entspannten Zustand und einem expandierten Zustand radial zu expandieren und sich zusammenzuziehen, damit die perkutane medizinische Vorrichtung (30) durch das Lumen gehen kann,
**gekennzeichnet durch** einen Geraderichtdraht (50), der verschiebbar in einem in der Wand (22) der Membran (20) ausgebildeten Durchgang angeordnet ist, wobei der Durchgang allgemein parallel zu dem Lumen ausgerichtet ist.

2. Medizinische Vorrichtungsanordnung nach Anspruch 1, wobei die Membran (20) dazu konfiguriert ist, es dem Lumen zu gestatten, radial zu einem zweiten inneren Durchmesser zu expandieren, der gleich dem maximalen äußeren Durchmesser oder größer als dieser ist.

3. Medizinische Vorrichtungsanordnung nach Anspruch 1, wobei die Membran (20) in einer im Wesentlichen stationären Position entlang dem Führungsdraht (10) bleibt, wenn die perkutane medizinische Vorrichtung (30) durch das Lumen geht.

4. Medizinische Vorrichtungsanordnung nach Anspruch 1, wobei die Membran (20) einen äußeren Durchmesser definiert, der kleiner als der maximale äußere Durchmesser der perkutanen medizinischen Vorrichtung (30) ist.

5. Medizinische Vorrichtungsanordnung nach Anspruch 1, wobei die Membran (20) eine erste Vielzahl von Fasern (42) aufweist, die jeweils parallel zum Lumen ausgerichtet sind.

6. Medizinische Vorrichtungsanordnung nach Anspruch 5, wobei die erste Vielzahl von Fasern (42) in der Wand (22) der Membran (20) eingebettet ist.

7. Medizinische Vorrichtungsanordnung nach Anspruch 5, wobei die erste Vielzahl von Fasern (42) an einer Fläche der Wand (22) der Membran (20) angeordnet ist.

8. Medizinische Vorrichtungsanordnung nach Anspruch 5, wobei die erste Vielzahl von Fasern (42) ein axiales Dehnen der Membran (20) entlang dem Lumen im Wesentlichen verhindert.

9. Medizinische Vorrichtungsanordnung nach Anspruch 1, wobei die Membran (20) eine zweite Vielzahl von Fasern (44) aufweist, die jeweils umfangsmäßig um das Lumen ausgerichtet sind.

10. Medizinische Vorrichtungsanordnung nach Anspruch 9, wobei die zweite Vielzahl von Fasern (44) in der Wand (22) der Membran (20) eingebettet ist.

11. Medizinische Vorrichtungsanordnung nach Anspruch 9, wobei die zweite Vielzahl von Fasern (44) an einer Fläche der Wand (22) der Membran (20) angeordnet ist.

12. Medizinische Vorrichtungsanordnung nach Anspruch 9, wobei die zweite Vielzahl von Fasern (44) die radiale Expansion der Membran und/oder des Lumens auf einen vorbestimmten Maximalwert begrenzt.

13. Medizinische Vorrichtungsanordnung nach Anspruch 1, wobei der Geraderichtdraht (50) axial zwischen einer relativ flexiblen ausgefahrenen Konfiguration und einer relativ starren zusammengezogenen Konfiguration betätigbar ist.

14. Medizinische Vorrichtungsanordnung nach Anspruch 1, wobei die Wand (22) eine schlüpfrige Beschichtung aufweist, die an einer inneren Fläche (24) davon angeordnet ist.

15. Medizinische Vorrichtungsanordnung nach Anspruch 1, wobei die perkutane medizinische Vorrichtung (30) aus Folgendem ausgewählt ist: eine Atherektomievorrichtung, eine Angioplastievorrichtung, ein Ballondilatationskatheter, eine distale Schutzvorrichtung, eine Emboliefiltervorrichtung, eine Valvektomievorrichtung, eine Valvuloplastie-vorrichtung, eine Stentzuführvorrichtung, eine Trans-Aortenklappenimplantationsvorrichtung, eine Ablationsvorrichtung, eine Fremdkörperentfernungsvorrichtung, ein Führungskatheter oder eine Führungshülse oder ein Diagnosekatheter.

## Revendications

1. Ensemble formant dispositif médical comprenant :
un fil-guide allongé (10) ;
une membrane tubulaire allongée à grande souplesse (20) disposée autour du fil-guide (10), la membrane (20) comportant une paroi (22) définissant une lumière à travers la membrane (20), la lumière présentant un premier diamètre intérieur ; et
un dispositif médical percutané (30) présentant un diamètre extérieur maximal supérieur au premier diamètre intérieur ;
la membrane (20) et la lumière étant configurées pour se dilater et se contracter radialement entre un état relâché et un état dilaté de façon à permettre au dispositif médical percutané (30) de passer à travers la lumière ;
**caractérisé par** un fil de redressement (50) disposé à coulissement dans un passage formé à l'intérieur de la paroi (22) de la membrane (20), le passage étant orienté de manière globalement parallèle à la lumière.

2. Ensemble formant dispositif médical selon la revendication 1, dans lequel la membrane (20) est configurée pour permettre à la lumière de se dilater radialement jusqu'à un second diamètre intérieur égal ou supérieur au diamètre extérieur maximal.

3. Ensemble formant dispositif médical selon la revendication 1, dans lequel la membrane (20) reste dans une position essentiellement stationnaire le long du fil-guide (10) à mesure que le dispositif médical percutané (30) est acheminé à travers la lumière.

4. Ensemble formant dispositif médical selon la revendication 1, dans lequel la membrane (20) définit un diamètre extérieur qui est inférieur au diamètre extérieur maximal du dispositif médical percutané (30).

5. Ensemble formant dispositif médical selon la revendication 1, dans lequel la membrane (20) comprend une première pluralité de fibres (42) orientées chacune de manière parallèle à la lumière.

6. Ensemble formant dispositif médical selon la revendication 5, dans lequel la première pluralité de fibres (42) est incorporée à l'intérieur de la paroi (22) de la membrane (20).

7. Ensemble formant dispositif médical selon la revendication 5, dans lequel la première pluralité de fibres (42) est disposée sur une surface de la paroi (22) de la membrane (20).

8. Ensemble formant dispositif médical selon la revendication 5, dans lequel la première pluralité de fibres (42) empêche essentiellement un étirement axial de la membrane (20) le long de la lumière.

9. Ensemble formant dispositif médical selon la revendication 1, dans lequel la membrane (20) comprend une seconde pluralité de fibres (44) orientées chacune de manière circonférentielle autour de la lumière.

10. Ensemble formant dispositif médical selon la revendication 9, dans lequel la seconde pluralité de fibres (44) est incorporée à l'intérieur de la paroi (22) de la membrane (20).

11. Ensemble formant dispositif médical selon la revendication 9, dans lequel la seconde pluralité de fibres (44) est disposée sur une surface de la paroi (22) de la membrane (20).

12. Ensemble formant dispositif médical selon la revendication 9, dans lequel la seconde pluralité de fibres (44) limite la dilatation radiale de la membrane et/ou de la lumière à une valeur maximale prédéterminée.

13. Ensemble formant dispositif médical selon la revendication 1, dans lequel le fil de redressement (50) peut être actionné axialement entre une configuration étendue relativement souple et une configuration contractée relativement rigide.

14. Ensemble formant dispositif médical selon la revendication 1, dans lequel la paroi (22) comprend un revêtement lubrifiant disposé sur une surface intérieure (24) de celle-ci.

15. Ensemble formant dispositif médical selon la revendication 1, dans lequel le dispositif médical percutané (30) est sélectionné parmi les suivants : un dispositif d'athérectomie, un dispositif d'angioplastie, un cathéter de dilatation à ballonnet, un dispositif de protection distal, un dispositif de filtration embolique, un dispositif de valvectomie, un dispositif de vavuloplastie, un dispositif de pose de stent, un dispositif d'implantation de valve transaortique, un dispositif d'ablation, un dispositif d'extraction d'objet, un cathéter ou une gaine de guidage ou un cathéter de diagnostic.
